# EUROPEAN PATENT APPLICATION

(11) **EP 0 673 623 A1**
(43) Date of publication of application: **27.09.1995**
(21) Application number: 94850046.7
(22) Date of filing: 24.03.1994
(51) Int. Cl.: A61B 6/14

(54) **Scanning layer forming radiography**

(71) Applicant: REGAM MEDICAL SYSTEMS AB, S-852 37 Sundsvall (SE)
(72) Inventor: Nelvig, Per, S-852 65 Sundsvall (SE)
(74) Representative: Mrazek, Werner

(57) **Abstract**

The present invention discloses a scanning digital radiographic system comprising an x-ray radiation source (15) with a collimator (14) including a first diaphragm with a narrow rectangular, vertically aligned, slit (22) creating a fan shaped x-ray beam directed toward the object, an xray image detector (19) having a total sensitive area approximately coincident with the cross-section area of the x-ray beam at the position of said xray image detector, an xray source-collimator assembly (15) being rigidly mechanically connected with said image detector into a collimated xray-source/detector assembly (18) which is rotatable around a vertically oriented axis (17) that is movable between any position in a horizontally defined plane (16). The combination of said collimated xray-source and a detector assembly consisting of an electronic detector array matching the cross section of the xray beam results that a secondary diaphragm positioned between the object to be imaged and the imaging detector array will no longer be necessary.

## Description

### Technical field

The present invention relates to x-ray imaging and more particularly to a scanning dental digital radiographic system.

### Prior art

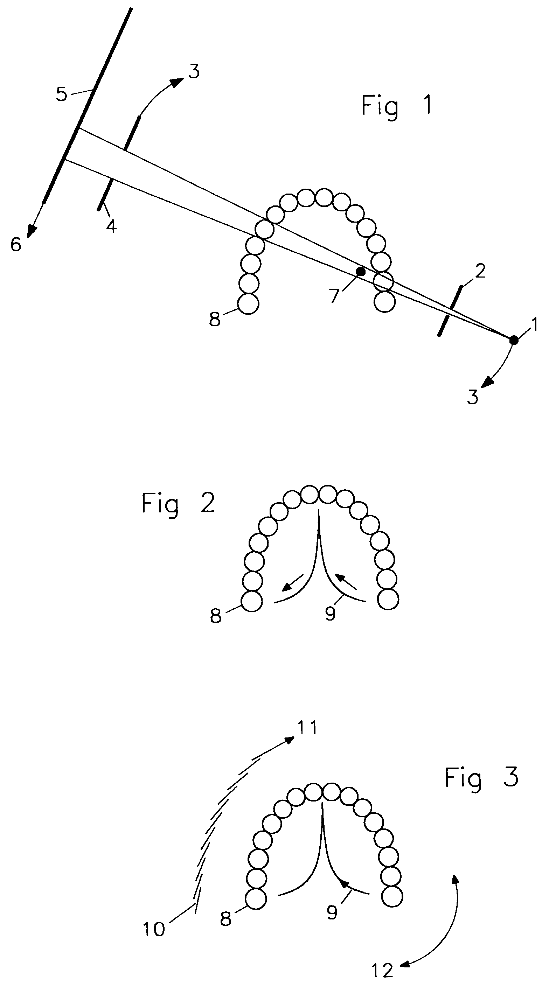

Since mid 1950:s a common method to produce overview x-ray images of teeth and jaws for use in dentistry is the so called panoramic x-ray method. Fig 1. demonstrates a typical prior art set up. Assuming the head of the patient is oriented in an upright position, the method uses a movable x-ray source 1 with the beam collimated to a narrow (3-6 mm) width in the horizontal dimension, and extended at least 12 cm in the vertical dimension. The collimation is done with a narrow slit in a piece of sheet metal made of an x-ray absorbing material placed at a suitable distance from the focal spot of the x-ray tube, but before the radiation reaches the patient. The plate 2 with the slit is often referred to as a primary diaphragm. On the opposing side of the patient, relative to the x-ray source, a second metal plate 3 with a narrow second slit, corresponding to the fan shaped beam is placed. This second piece of sheet metal with the second slit is often referred to as a secondary diaphragm. The slits and the x-ray source with it's collimator are rigidly attached to each other. The film 5 is placed further away from the x-ray source, in the direction of the x-ray beam, at a position behind the second slit. The function of the secondary diaphragm is mainly to protect those parts of the film covered by it from being exposed by secondary (scattered) radiation. A second function is to make the beam more well defined when it hits the film. During the exposure, usually taking 15 - 20 seconds for an ordinary panoramic x-ray, 30 cm wide, the arrangement of x-ray source and slit is rotated in a direction 3 around the head of the patient in a controlled manner so that a rotational center 7 of the imaging system will be situated within the head of the patient. At the same time, the imaging system will move relative to the film which will be exposed by radiation through the patients head and the second slit, piece by piece, until all of the film has been exposed during the time the imaging system was rotated around its rotational center 7.

By controlling the film speed relative to the object, the x-ray beam and the projection geometry with the rotational center assumed as a "virtual focus" that is moved during the exposure, it can be demonstrated that only a predetermined layer in the object is sharply depicted. In order to bring this sharp layer to coincide with the dental arches and to compensate for magnification variations in the vertical and horizontal dimensions, a rather complex motion sequence is required, where the vertically oriented rotational axis 7 is not fixed within the patients head but will be moving along a continuous path in the horizontal plane approximately parallel to the occlusal plane according to fig. 2.

A good reference for a popular technical description of conventional panoramic radiography is found in chapter 2, "Theory of Rotational Panoramic Radiography.", second edition of "Panoramic radiology" by Olaf Langland, Robert Langlais, Doss McDavid, Angelo DelBalso, printed by Lea & Febiger, Philadelphia 1989.

The traditional method can be viewed as a method to add an indefinite number of images, each of them with indefinitely low exposure, in size corresponding to the slit, directly on the analog film medium, simultaneously moving the film past the slit as described above. However, the method would work equally well using a large but finite number of discrete steps both in the x-ray source/slit assembly motion and in the motion of the film.

It is obvious that the described method could be modified to utilize for instance semiconductor detectors replacing the film. Three examples of relevant patents are the U. S. Patents No. 4,878,234, 4,823,369 and 5,018,177.

The first patent document U.S. Patent No. 4,878,234 deals with a method and an apparatus to use one or several CCD (Charge Coupled Device) detectors for both detecting the image information and for simulation of the film motion directly in the imaging area by, since the early development of CCD:s, the well known method named TDI (Time Delayed Integration) wherein the individual pixel charges can be moved to simulate film motion by the clocking sequence of the detector. This method has the same limitations as the film method regarding the necessary complex motion pattern to adjust the horizontal magnification and to determine the position of the sharp layer. An obvious drawback is that the position of this layer has to be pre-determined, before the exposure. The image is digitized during the exposure sequence and immediately subsequently presented on a video monitor.

The second patent publication U.S. Patent No. 4,823,369 deals with a method and an apparatus to capture a large number of overlapping images using a semiconductor image detector with a dimension corresponding to the above described slit, in real time postprocessing each of the images by adding pixels adjacent to each other to create fewer columns of image data to reduce the amount of data. Each of the resulting combined columns would correspond to a readout register of a detector as described in the previous U.S. Patent No. 4,878,234 or to an ordinary image column with reduced spatial resolution. Image data from the set of columns after postprocessing is stored at separate locations in a large image memory. A reconstruction of panoramic images is performed after the completion of the exposure by adding the images stored in memory to another memory, slightly positionally shifted relative to each other, until one or more resulting images with a size corresponding to approximately 12.5 x 30 cm has been totally covered using the sums of a large number of smaller images.

This method reduces the spatial resolution or restricts the subsequent reconstruction to a restricted number of predetermined layers depending on how many groups of image column data that will be created during the data reduction.

It should be noted that in both above disclosed patent documents a secondary diaphragm is included as a necessary requirement in all the independent claims. However, a secondary diaphragm would be completely unnecessary when a large area film is replaced by a detector which only picks up radiation falling within the borders of the sensitive area.

The third document U.S. Patent No. 5,018,177 discloses a method and an apparatus for producing among other possible projections, panoramic radiograms, using one single, vertically oriented line detector, consisting of a number of pixel detectors. This document mainly deals with the idea to divide the complete predetermined scanning period into a series of time intervals which are a function of the elapsed time within the total scanning time period. The signal created by radiation in the detector is integrated during each time interval. At the end of each time interval the analog signal from the detector is converted to digital data. By means of data processing a complete two dimensional radiogram is produced from the set of column data from the detector. The disclosure also deals with a way to calculate the described time intervals from a given projection geometry and a given predetermined scanning time interval to obtain a panoramic radiogram.

The method has the general drawback for panoramic radiography, compared to the traditional method and the two other cited disclosures, that there is no creation of a layer. All layers within the object will be represented equally sharp in the image. As a consequence, significant overprojection from for dentistry irrelevant structures such as the spinal column of the neck, is interfering with the relevant information. In the traditional film method and the methods described under the first two disclosures mentioned, the irrelevant objects, positioned outside the sharp layer, are blurred to the extent that they don't interfere with the relevant image information.

### Short description of the invention

An object of the present invention is to provide a scanning dental digital radiographic system being more elaborate but having less elements compared to prior art.

According to a first object of the present invention is disclosed a scanning digital radiographic system comprising an x-ray radiation source with a collimator containing a first diaphragm including a narrow rectangular, preferably vertically aligned, slit creating a fan shaped x-ray beam directed toward the object, an xray image detector having a total sensitive area approximately coincident with the cross-section area of the x-ray beam at the position of said xray image detector, an xray source-collimator assembly being rigidly mechanically connected with said image detector into a collimated xray-source/detector assembly which is rotatable around a vertically oriented axis that is movable between any position in a horizontally define plane whereby said collimated xray-source/detector assembly will be lacking a secondary diaphragm positioned between the object to be imaged and imaging detector.

According to a second object of the present invention the collimated xray source/detector assembly is moved relative to the object during exposure such that sequentially different parts of the object to be imaged are scanned, continuously or discretely collecting image information from the detector, whereby at the end of a scan a set of images is obtained so that each point in the object is represented in one or several of the added up images, two or more images then representing different projection geometries of the object to be imaged.

According to a third object of the present invention the imaging detector comprises one or more CCD-detectors combined with an optically coupled energy converter converting from xray photon energy into light photon energy, the CCD-detector having the parallel shiftable columns oriented parallel to the plane of motion for the collimated xray source-collimator/detector assembly and the electric charge containing image information collected being shifted pixel by pixel during the scan with a speed determined such that the projection of an object detail in a pre-determined layer of the object coincides with the position of the previously integrated image information electric charge of the same object detail collected earlier but with another projection geometry, thereby during the scan obtaining the sum of image information with several different projections of an object detail when the electric charge is read-out.

According to a fourth object of the present invention the collimated xray-source/detector assembly is rotated around the head of a patient during the exposure, and the rotational axis is moved constantly or in discrete steps along a path in a plane approximately parallel to the occlusal plane, simulations of ordinary dental panoramic radiograms are reconstructed by digitally adding up image information from the set of images representing each point in the object in such a way that each point in the object positioned in the desired sharp layer will be added into the same memory location of a memory storing the finally reconstructed radiogram.

According to a fifth object of the present invention the collimated xray-source/detector assembly is rotated around the head of a patient during the exposure, and the rotation axis is moved constantly or in discrete steps along a path in a plane approximately parallel to the occlusal plane, simulations of ordinary dental panoramic radiograms can be reconstructed by directly adding up image information electric charges in one or more CCD-detectors, moving these electric charges by a general clocking method such that the projected image of a point within the sharp layer of the object will positionally coincide with the previously collected electric charge representing the same point in the object.

### Short description of the drawings

The invention will be described by preferred embodiments to be contemplated with reference to the accompanying drawings wherein like reference numerals are used throughout to designate like parts. In the drawings:
- Fig. 1: shows a traditional panoramic narrow slit rotational radiography with film,
- Fig. 2: shows dental arch with a motion path of the rotational axis during an exposure sequence,
- Fig. 3: shows a motion of the detector during a part of an exposure sequence from one position to another and at the same time the xray focal spot moves along the path indicated by the arrow and the rotational axis is moved along a path according to fig. 2,
- Fig. 4: shows an adding up of vertically oriented, horizontally shifted, narrow xray images creating an added up radiogram covering a larger area than a single image,
- Fig. 5: demonstrates in a plane view a preferred embodiment according to the present invention,
- Fig. 6: demonstrates in a view elevation a preferred embodiment of the present invention,
- Fig. 7: demonstrates in a side view a preferred embodiment of the present invention,

### Description of a preferred embodiment

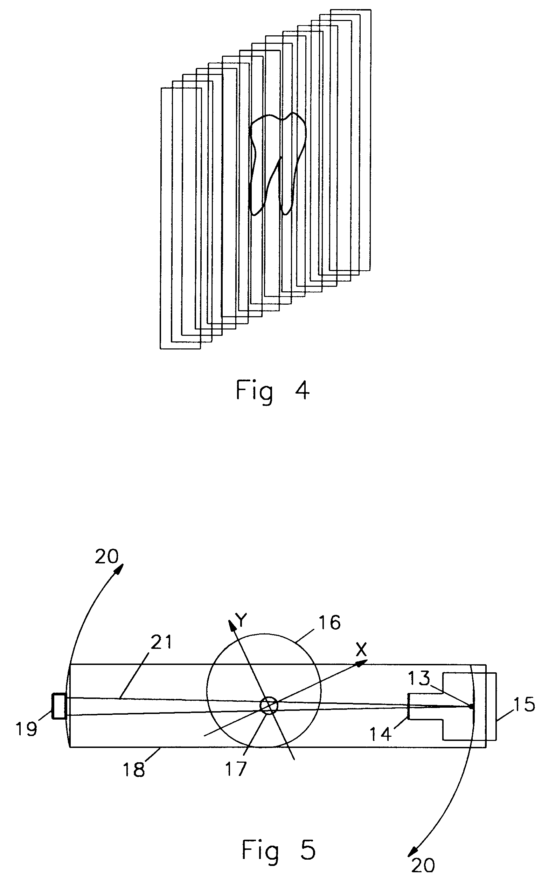

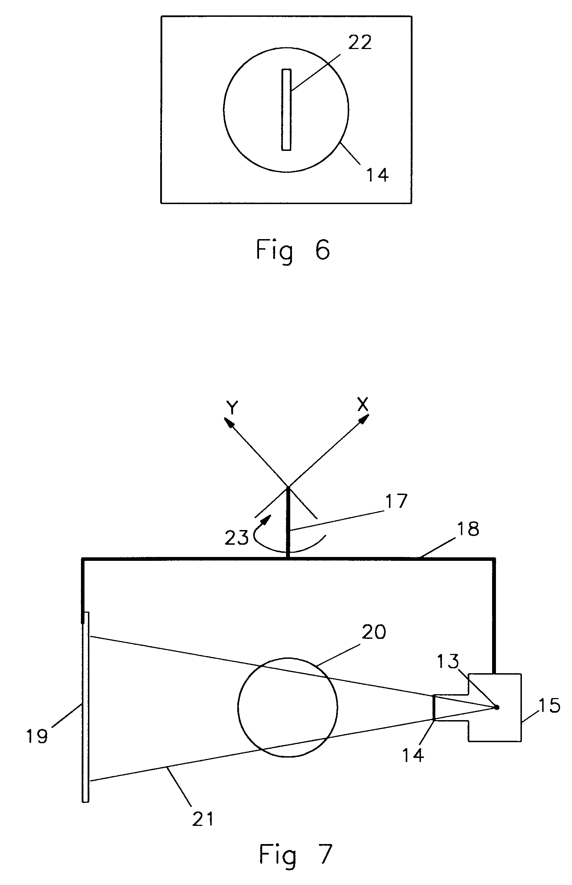

A illustrative embodiment of the current invention comprises according to Fig. 5 a case 15 containing an xray-source 13 and in rigid mechanical connection with a rectangular imaging detector 19 having its long sides vertically oriented. This xray-source/detector assembly 18 is rotatable around a vertically oriented axis 17 that is movable between any positions within a horizontal plane 16. Close to the x-ray source 13 in the case 15 there is a collimator with a primary diaphragm 14 provided with a slit 22 (Fig. 6), which together serves to direct the xray-beam to the detector and to adapt the cross section of beam at the detector to approximately match the active area of the detector 19. The main purpose for matching the beam area to the detector area is to avoid more radiation to the patient than what is required for the acquirement of the images. A second reason for limiting the beam area is to reduce the amount of secondary radiation induced in the object. Excess secondary radiation decreases the image quality. There will be needed no secondary diaphragm placed in front of the detector in this embodiment according to the present invention since secondary radiation with a direction falling outside the sensitive area of the detector will not affect the image quality.

If the desired projections are simulations of traditional panoramic radiograms of the jaws of a patient the function would be described as follows:

The patient is placed with the head in a fixed and upright position between the xray source 13 and the detector 19 during the exposure sequence. During the exposure sequence the xray-source/detector assembly 18 is rotated around the object along a path that is partially or completely circumscribing the object while the rotational center 17 is moved along a path in the horizontal plane 16. During the period of motion, the xray-source 15 is emitting xrays in the shape of a beam directed towards the jaws of the patient. The fraction of the xray flux not absorbed in the object, subsequently hits the detector 19. During the exposure and motion sequence, a large number of images are captured by the detector. The image data of each image is immediately converted to digital data and subsequently stored in a digital memory, each image having it's own storage location in memory. After the completion of the exposure/motion sequence, different panoramic radiograms with different layer positions may be reconstructed by methods well known by a person skilled in the art. The layer positioning may be adapted to exactly fit the individual anatomy of the jaws.

For the final reconstruction, it is required that each point in the finally reconstructed image must be represented in two or more positionally shifted images, otherwise there will be no layer formation. Preferably, information from five or more partially overlapping images is to be used for the reconstruction of each point in the final radiogram.

In a less memory consuming embodiment than the one sketched above the final radiogram of one pre-defined layer may gradually be reconstructed during the scan sequence. Each portion of the final radiogram would in this case be reconstructed as soon as a sufficient number of overlapping images has been acquired. In this case a temporary storage memory capable to contain only, for instance, five images will be needed to maintain input data for the reconstruction. When a new image is captured, it will simply replace the oldest previously stored image.

In an even less memory consuming embodiment the above mentioned TDI method can be used to immediately add information from continuously or stepwise changed projections directly into a CCD detector. As new projections are formed, the previously collected image information containing charges in the CCD are spatially shifted, using known clocking methods, so that the new charges containing image information for the actual projection is added to the existing charges in a way that creates a sharp layer simulating a panoramic radiogram.

To fulfill the requirements of a good spatial resolution, the integration time for each image must either be small compared to the motion of the rotational axis to keep the motional unsharpness low or the TDI-method may be used during the integration period to compensate for the motion of the rotational axis.

The effective pixel size is selected to fit the required resolution in the final radiogram. To obtain a resolution of two line pairs per mm an effective pixel size smaller than approximately 250 by 250 pm will be needed. The physical pixel size in the detector can be smaller and through post-processing, before or after storage into the reconstruction memory, information from several smaller pixels can be added to form the required pixel size in the reconstructed radiogram.

Any image detector that can acquire image data at the required speed may be selected to constitute the image detector used in the preferred embodiment. In a further embodiment to enhance the xray response, the detector will be combined with an intensifying screen of a material capable to convert the energy of the xray photons to an energy level better adapted to the detector. Of course the detector may as well be divided into separate sections to facilitate easier manufacturing.

Above embodiments further may be modified by changing the motion sequence in relation to the exposure sequence, thereby enabling an optional imaging of optional plane or curved layers or enabling non-layered images.

## Claims

1. A scanning digital radiographic system comprising:
an x-ray radiation source (15) including a collimator (14) and only one diaphragm having a narrow, rectangular slit (22) creating a fan shaped x-ray beam directed toward the object,
an xray image detector (19) having a total sensitive area approximately coincident with the cross-section area of the x-ray beam at the position of said xray image detector,
an xray source-collimator assembly (15) being rigidly mechanically connected with said image detector (19) into a collimated xray-source/detector assembly (18) which is rotatable around a vertically oriented axis (17) that is movable between any position within a horizontally defined plane (16).

2. The system according to claim 1, wherein said collimated xray source/detector assembly (18) is moved relative to the object during exposure such that sequentially different parts of the object are scanned, continuously or discretely collecting image information from the detector (19), whereby at the end of a scan a set of images is obtained so that each point in the object is represented in at least one of the added up images.

3. The system according to claim 1, wherein said collimated xray-source/detector assembly (18) is moved relative to the object during exposure such that sequentially different parts of the object are scanned, continuously or discretely collecting image information from the detector, whereby at the end of a scan a set of images is obtained so that each point in the object is represented in at least two or preferably several of the added up images, where the two or more images represent different projection geometries of the object.

4. The system according to claim 1, wherein said detector (19) comprises one or more CCD-detectors combined with an optically coupled energy converter converting from xray photon energy into light photon energy, the CCD-detector having the parallel shiftable columns oriented parallel to the plane of motion for said collimated xray source-collimator/detector assembly (18) and the electric charge containing image information collected being shifted pixel by pixel during the scan with a speed determined such that the projection of an object detail in a pre-determined layer of the object coincides with the position of the previously integrated image information electric charge of the same object detail collected earlier but with another projection geometry, thereby during the scan obtaining the sum of image information with several different projections of an object detail when the electric charge is read-out.

5. The system according to claim 1, wherein said collimated xray-source/detector assembly (18) is rotated around the head of a patient during the exposure, and the rotational axis (17) is moved constantly or in discrete steps along a path (9) in a plane (16) approximately parallel to the occlusal plane, simulations of ordinary dental panoramic radiograms are reconstructed by digitally adding up image information from the set of images representing each point in the object in such a way that each point in the object positioned in the desired sharp layer will be added into the same memory location of a memory storing the finally reconstructed radiogram.

6. The system according to claim 1, wherein said collimated xray-source/detector assembly (18) is rotated around the head of a patient during the exposure, and the rotation axis is moved constantly or in discrete steps along a path in a plane approximately parallel to the occlusal plane, simulations of ordinary dental panoramic radiograms can be reconstructed by directly adding up image information electric charges in one or more CCD-detectors, moving these electric charges by a general clocking method such that the projected image of a point within the sharp layer of the object will positionally coincide with the previously collected electric charge representing the same point in the object.
